(19) Europäisches Patentamt
European Patent Office
Office européen des brevets

(11) EP 4 390 378 A1

(12) # EUROPEAN PATENT APPLICATION

published in accordance with Art. 153(4) EPC

(43) Date of publication:
**26.06.2024 Bulletin 2024/26**

(21) Application number: **22858451.2**

(22) Date of filing: **15.08.2022**

(51) International Patent Classification (IPC):
***G01N 21/65*** *(2006.01)* ***G01N 21/27*** *(2006.01)*
***G01N 21/64*** *(2006.01)*

(52) Cooperative Patent Classification (CPC):
**G01N 21/27; G01N 21/64; G01N 21/65**

(86) International application number:
**PCT/JP2022/030901**

(87) International publication number:
**WO 2023/022132 (23.02.2023 Gazette 2023/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.08.2021 JP 2021132922**

(71) Applicant: **RIKEN**
**Wako-shi, Saitama 351-0198 (JP)**

(72) Inventors:
- **WADA, Satoshi**
**Wako-shi, Saitama 351-0198 (JP)**
- **OGAWA, Takayo**
**Wako-shi, Saitama 351-0198 (JP)**
- **TAKETANI, Akinori**
**Wako-shi, Saitama 351-0198 (JP)**

(74) Representative: **Patentanwälte Olbricht Buchhold Keulertz**
**Partnerschaft mbB**
**Neue Mainzer Straße 75**
**60311 Frankfurt am Main (DE)**

(54) **COMPONENT ANALYZING DEVICE, AND COMPONENT ANALYZING METHOD**

(57) Provided is a component analyzing device 1 including: a projection unit 11 which projects light on an object; a measurement unit 12 which detects reflected light, scattered light, or emitted light from the object and measures a content of a related substance, which is contained together with a target substance in the object, from a detection result; and an estimation unit 13 which estimates a content of the target substance from a measurement result of the content of the related substance, based on a quantitative relationship between the target substance and the related substance derived from a reaction equation system based on a biosynthetic metabolic pathway of the object.

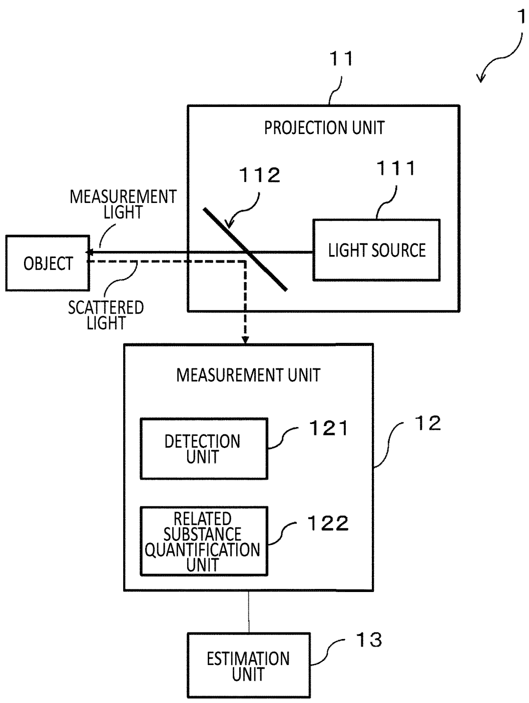


FIG.1

EP 4 390 378 A1

## Description

BACKGROUND

1. TECHNICAL FIELD

The present invention relates to a component analyzing device and a component analyzing method for non-invasively analyzing a content of a component in a plant.

2. RELATED ART

Functional components contained in plants such as fruits and grains have attracted attention. For example, procyanidin contained in apple is a kind of flavonoid, and has a strong antioxidant effect and various other health effects. Therefore, the food containing the procyanidin can be labeled as a food for specified health uses or a functional labeling food. In this regard, it is desired to measure the content of a function-generating component contained in a plant by a non-invasive measurement method such as a spectroscopic spectrum method described in Patent Document 1.
Patent Document 1: Japanese Patent No. 6323749

PROBLEMS TO BE SOLVED

However, since functional components, such as procyanidin, contained in plants are present in trace amounts and highly reactive, and are difficult to be non-invasively measured, an invasive measurement such as a chromatography method is required. A method for non-invasively measuring a trace amount of a highly reactive component has not been established.

General Disclosure

(Item 1)

A component analyzing device may include a projection unit which projects light on an object.
The component analyzing device may include a measurement unit which detects reflected light, scattered light, or emitted light from the object and measures a content of a related substance, which is contained together with a target substance in the object, from a detection result.
The component analyzing device may include an estimation unit which estimates a content of the target substance from a measurement result of the content of the related substance, based on a quantitative relationship between the target substance and the related substance derived from a reaction equation system based on a biosynthetic metabolic pathway of the object.

(Item 2)

The quantitative relationship may be derived based on a reaction equation system and a reaction rate constant regarding a metabolic pathway leading to each of the target substance and the related substance from a precursor substance common to the target substance and the related substance in the biosynthetic metabolic pathway of the object.

(Item 3)

The quantitative relationship may be derived by first-order approximation of the reaction equation system regarding the metabolic pathway leading to each of the target substance and the related substance from the precursor substance.

(Item 4)

The related substance may be selected from substances that at least partially have a common metabolic pathway with the target substance in the biosynthetic metabolic pathway of the object.

(Item 5)

The related substance may be selected from substances, which allow measurement using light and have a large content, among substances contained in the object.

(Item 6)

A metabolic pathway which generates the target substance and a metabolic pathway which generates the related substance may stably branch from a common metabolic pathway in the biosynthetic metabolic pathway of the object.

(Item 7)

The estimation unit may further verify an estimated content of the target substance by comparison with a measured content of the target substance.

(Item 8)

the object may be a plant including a fruit and a vegetable,

The target substance may be procyanidin, and

the related substance may be a carotenoid.

(Item 9)

A component analyzing method may include projecting light on an object.
The component analyzing apparatus may include detecting reflected light, scattered light, or emitted light from the object and measuring a content of a related substance, which is contained together with a target substance in the object, from a detection result.
The component analyzing apparatus may include estimating a content of the target substance from a measurement result of the content of the related substance, based on a quantitative relationship between the target substance and the related substance derived from a reaction equation system based on a biosynthetic metabolic pathway of the object.
The summary clause does not necessarily describe all necessary features of the embodiments of the present invention. The present invention may also be a sub-combination of the features described above.

BRIEF DESCRIPTION OF THE DRAWINGS

Fig. 1 shows a configuration of a component analyzing device according to the present embodiment.

Fig. 2 shows the 128 Raman spectrum measurement results of an apple preprocessed by the related substance quantification unit.

Fig. 3 shows the average of the Raman spectrum measurement results of the apple preprocessed by the related substance quantification unit.

Fig. 4 shows a biosynthetic metabolic pathway of the apple.

Fig. 5 shows a part of a simplified secondary metabolic pathway of the apple.

Fig. 6 shows a comparison between a procyanidin content (predicted value) analyzed by the component analyzing device according to the present embodiment and a procyanidin content (measured value) measured by chromatography.

Fig. 7 shows a flow of a component analyzing method according to the present embodiment.

DESCRIPTION OF EXEMPLARY EMBODIMENTS

Hereinafter, the present invention will be described through embodiments of the invention, but the following embodiments do not limit the invention according to the claims. In addition, not all of the combinations of features described in the embodiments are imperative to the solving means of the invention.

Fig. 1 shows a configuration of a component analyzing device 1 according to the present embodiment. The component analyzing device 1 is a device which analyzes a component (also referred to as a substance) contained in a plant and quantifies the content thereof, and in particular, analyzes a component which is difficult to be non-invasively measured, for example, since only a trace amount of the component is contained in the plant, the absorption or reflection spectrum of the component is superimposed on a spectrum of another substance in the plant, or the content of the component is changed by a measurement action due to high reactivity. Here, a plant to be analyzed by the component analyzing device 1 is also simply referred to as an object. The substance in the object quantified by the component analyzing device 1 is also simply referred to as a target substance. In the present embodiment, as an example, the object is an apple, and the target substance is procyanidin.

In particular, the component analyzing device 1 according to the present embodiment directly measures the related substance contained in the object together with the target substance by using light, and estimates the content of the target substance from the content of the related substance, thereby non-invasively analyzing and quantifying the target substance in the object. As the related substance, it is desirable to select a substance which can be measured by using light, has a large content, and has a quantitative relationship with the target substance in the content. Here, the quantitative relationship is a relationship derived from a reaction equation system based on a biosynthetic metabolic pathway of the object, and refers to a relationship which is observed between the content of the target substance and the content of the related substance due to partial commonality of the metabolic pathway or the like. Accordingly, it is possible to estimate, with high accuracy, the content of the target substance which is difficult to be non-invasively measured. In the present embodiment, the related substance is β carotene which has a quantitative relationship with procyanidin as the target substance, is easily measured by using light, and is a kind of pigment having a high content in an apple as the object. Details of the quantitative relationship will be described later.

The component analyzing device 1 includes a projection unit 11, a measurement unit 12, and an estimation unit 13.

The projection unit 11 is a unit which irradiates the object with measurement light, and includes a light source 111 and a beam splitter 112.

The light source 111 generates measurement light and emits the measurement light toward the object. In the present embodiment, since β carotene is a related substance, a semiconductor laser which generates near-infrared laser light having a wavelength of 785 nm is employed as an example of the light source 111. The measurement light is not limited to near-infrared light (wavelength: 780 to 2500 nm), and visible light (380 to 780 nm), ultraviolet light (380 nm or less), or the like may be used according to the emission spectrum of the related substance.

The beam splitter 112 is an optical member which transmits the measurement light emitted from the light source 111 and reflects the scattered light scattered by the object toward the measurement unit 12. As the beam splitter 112, a half mirror, a polarization beam splitter, or the like can be employed.

The measurement unit 12 is a unit which detects the reflected light reflected from the object and measures, from the detection result, the content of the related substance contained together with the target substance in the object. Here, the measurement unit 12 may detect not only the reflected light from the object but also the scattered light scattered from the object or the light emitted by the object. In addition, the Raman scattered light scattered from the object may be measured by irradiation with measurement light. The measurement unit 12 includes a detection unit 121 and a related substance quantification unit 122.

The detection unit 121 includes a spectrometer which spectrally detects the scattered light scattered from the object. Here, in order to detect the Raman scattered light generated by irradiating the object with measurement light having a wavelength of 785 nm, the detection unit 121 detects the scattered light, for example, in a wave number range of 800 to 1600 $cm^{-1}$ on a longer wavelength side than the measurement light. The spectrum data of the detected scattered light is transferred to the related substance quantification unit 122.

The related substance quantification unit 122 analyzes the spectrum data of the scattered light detected by the detection unit 121 and quantifies the related substance contained in the object. The related substance quantification unit 122 has a computer terminal, and expresses an analysis function by causing the computer terminal to execute a dedicated program.

The related substance quantification unit 122 extracts the spectrum of the scattered light Raman-scattered from the object, that is, the Raman spectrum by preprocessing the spectrum data of the scattered light transferred from the detection unit 121. The related substance quantification unit 122 first removes, from the spectrum data of the scattered light, a spectrum in a state where the object is not irradiated with the measurement light, that is, the noise derived from a spectroscopic device. Next, a baseline is corrected by using polynomial approximation, so that the noise derived from the fluorescence generated from the object by irradiation with the measurement light is removed from the spectrum data from which the noise derived from the spectroscopic device has been removed. Next, periodic interference fringes are removed by performing Fourier transform on the spectrum data from which the noise derived from the fluorescence has been removed. Finally, a spectral intensity is corrected with respect to the spectrum data from which the interference fringes have been removed, thereby obtaining the Raman spectrum of the object.

Fig. 2 shows a Raman spectrum of an apple obtained by the irradiation of the projection unit 11 on the apple with measurement light having a wavelength of 785 nm, the detection performed by the detection unit 121 of the measurement unit 12, and further the preprocessing performed by the related substance quantification unit 122. The Raman spectrum is indicated by the spectral intensity (arbitrary unit) with respect to a Raman shift ($cm^{-1}$). The Raman shift ($cm^{-1}$) is a value indicating how much the wavelength of the scattered light deviates from the wavelength of the measurement light, and the unit thereof is a wave number. In this example, measurement is performed 128 times and the Raman spectra obtained therefrom are shown in an overlapping manner.

Fig. 3 shows the average of the 128 Raman spectra shown in Fig. 2. A region A surrounded by a square with a solid line indicates a Raman spectral component derived from β carotene (also referred to simply as a Raman component), and a region B surrounded by a square with a broken line indicates a Raman component derived from procyanidin. In the region B, the spectral intensity is small, and a significant signal cannot be observed with respect to the noise level. Therefore, it can be seen that the procyanidin contained in the apple is in a trace amount, and it is difficult to detect the procyanidin by spectroscopic analysis. On the other hand, in the region A, the spectral intensity is large, and a significant signal peak can be observed with respect to the noise level. Therefore, it can be seen that the content of β carotene can be quantified by spectroscopic analysis.

Further, the related substance quantification unit 122 decomposes the Raman component derived from β carotene from the averaged Raman spectrum shown in FIG. 3 by means of multivariate analysis or the like, and multiplies the intensity of the Raman component derived from β carotene by a conversion coefficient to quantify the content of β carotene. Here, for example, by calculating the intensity of the Raman component of the related substance for some objects and further separately performing destructive measurement by the chromatography method to measure the content of the related substance, the conversion coefficient can be derived from a ratio between the intensity of the Raman component of the related substance and the measured value of the content by the destructive measurement.

The estimation unit 13 estimates the content of the target substance from the measurement result of the content of the related substance by the measurement unit 12, based on a quantitative relationship between the target substance and the related substance. The estimation unit 13 has a computer terminal, and expresses an estimation function by causing the computer terminal to execute a dedicated program. Note that the function of the estimation unit 13 may be expressed by the same computer terminal together with the related substance quantification unit 122. Here, the quantitative relationship is derived based on a reaction equation system regarding each of a metabolic pathway leading to the target substance and a metabolic pathway leading to the related substance in the biosynthetic metabolic pathway of the object.

In order to derive a significant quantitative relationship, it is preferable to select the related substance so that, in the biosynthetic metabolic pathway of the object, the metabolic pathway leading to the target substance and the metabolic pathway leading to the related substance have a common precursor substance. In addition, it is preferable to select the related substance so that the metabolic pathway leading to the target substance and the metabolic pathway leading to the related substance branch from a common metabolic pathway. By selecting the related substance in this way, it is possible to select the related substance strongly correlated with the target substance in the content.

Fig. 4 shows a biosynthetic metabolic pathway of the apple. The biosynthetic metabolic pathway includes a primary metabolic pathway and a secondary metabolic pathway. The primary metabolic pathway is a metabolic pathway for generating a substance essential for life activities, and includes a series of reactions in which, in apples, glucose is generated from carbon dioxide and water by photosynthesis (not shown), glucose reacts to generate PEP, PEP reacts to generate pyruvic acid, and the pyruvic acid reacts to generate MEP or the like.

The secondary metabolic pathway is a metabolic pathway for generating a substance not essential for life activities, and PEP reacts to generate shikimic acid, shikimic acid reacts to generate an alkanoid and a phenylpropanoid, and the phenylpropanoid react to generate a flavonoid. The flavonoid contains procyanidin which is the target substance in the present embodiment. Here, regarding the reaction for generating a flavonoid from a phenylpropanoid, there is a report that UV-B acts on epidermal cells to promote the generation of a flavonoid (H. Higashio, Proceedings of vegetable and tea science (2004)). In the secondary metabolic pathway, pyruvic acid reacts to generate acetyl CoA, the acetyl CoA reacts to generate mevalonic acid, and the mevalonic acid reacts to generate a carotenoid. The carotenoid contains β carotene which is the related substance of the present embodiment. Here, regarding the reaction in which acetyl CoA is generated from pyruvic acid, there is a report that the activity of pyruvic acid kinase is increased by irradiation with blue light (W. Kowallik et al., Planta, 128, 11 (1976)). Regarding the reaction in which a carotenoid is generated from mevalonic acid, there is a report that the expression of a carotenoid is amplified at a mRNA level by light irradiation (Masayasu M. et al., Hort.Res. (Japan) 14(4): 391 to 396, (2015)). Further, in the secondary metabolic pathway, MEP or the like and mevalonic acid react to generate a terpenoid. In this way, the secondary metabolic pathway includes a plurality of series of reactions and generally generates more diverse substances than in the primary metabolic pathway. In the secondary metabolic pathway of plants, carotenoid generation and flavonoid generation are synergistically promoted to react by light. Therefore, it is considered that there is a strong correlation between the contents of both.

The biosynthetic metabolism in the object can be expressed in a general form of a chemical reaction equation system representing that all the compounds i contained in the object are generated by the reaction of the respective precursor substances j, and disappear with the reaction of the subsequent substance j.

(Expression 1)

$$\frac{d[i]}{dt} = \sum_{j} \mathrm{kji} \times [\mathrm{j}] - \sum_{j} \mathrm{kij} \times [\mathrm{i}]$$

Here, i and j are indexes representing compounds contained in the object, and (i) is the content (concentration) of the compound i. kji is a reaction rate constant of a chemical reaction in which the compound j reacts to generate the compound i, and is given as finite when there is a metabolic pathway from the compound j to the compound i, and zero when there is no metabolic pathway, t is time at which the biosynthetic metabolism has progressed in the object. The general solution (i) = (i) (kji, kij, (j), t;j $\neq$ i) of Expression 1 is expressed as a function of the reaction rate constants kji and kij and the concentration (j) (where j is all but i). Here, the function (i) (kji, kij, (j), t;j $\neq$ i) may be expressed by an analytical function or may be expressed numerically by a numerical solution. Therefore, in the case of the target substance o and the related substance p, the quantitative relationship (o)/(p) thereof is given as (o)/(p) = (o) (kjo, koj, (j), $\tau$; j $\neq$ o)/(p) (kjp, kpj, (j), $\tau$; j $\neq$ p). The quantitative relationship (o)/(p) gives the value of the concentration (o) of the target substance o corresponding to the value of the concentration (p) of the related substance p, by using, as a medium parameter, a concentration (q) of a common precursor substance q of the target substance o and the related substance p, a concentration (r) of a starting substance r of biosynthetic metabolism in the object, or a concentration (s) of another substance s. The quantitative relationship (o)/(p) can be regarded as a function related to the concentration (p) of the related substance p which gives the concentration (o) of the target substance o, and can express not only a linear behavior of the concentration (o) with respect to the concentration (p) but also a nonlinear behavior.

Here, although the quantitative relationship (o)/(p) is also a function of time t, but biosynthetic metabolism of plants generally stops due to stop of water supply, a change in temperature of a growth environment, completion of growth and harvest, or the like, and thus a period $\tau$ until then can be substituted into a time variable t. Note that in the case of apples, $\tau$ can be set to a growth period, that is, about six months from fruiting to harvesting of fruits.

Note that in order to express the chemical reaction in the metabolic pathway in more detail, the reaction rate constant kji is not limited to a constant, and may be a function of environmental factors such as a temperature, a water amount, a light intensity, and a salt concentration.

The biosynthetic metabolism of the object can also be simplified to transform the general form of Expression 1 into a simpler form. For example, when it can be simplified that in the metabolic pathway, a compound A reacts to generate a compound B, the compound B reacts to generate a compound C, and the compound C reacts to produce a compound D in a chain manner, the chemical reaction equation system is expressed as follows.

(Expression 2)

$$\frac{d[B]}{dt} = \mathrm{k'1} \times [\mathrm{A}] - \mathrm{k'2} \times [\mathrm{B}]$$

$$\frac{d[C]}{dt} = \mathrm{k'2} \times [\mathrm{B}] - \mathrm{k'3} \times [\mathrm{C}]$$

$$\frac{d[D]}{dt} = \mathrm{k'3} \times [\mathrm{C}] - \mathrm{k'4} \times [\mathrm{D}]$$

Here, (A) to (D) are the concentrations of the compounds A to D, k'1 to k'4 are the reaction rate constants of the compounds A to D, respectively, and t is the time when biosynthetic metabolism has progressed in the object. Note that the content of the compound A was sufficiently large and can be regarded as constant. Expression 2 expresses a relationship in which due to three consecutive chemical reactions, a precursor substance reacts to generate each compound, and at the same time, the compound reacts to disappear.

Further, depending on the selection of the target substance o and the related substance p, for example, when the generation amount of each product is small with respect to the precursor substances in the biosynthetic metabolic pathway, the concentration of each compound can be derived by approximating as follows, ignoring the decrease in concentration of the precursor substance.

(Expression 3)

$$\frac{d[B]}{dt} = k'1 \times [A], \qquad [B] = \int^{\tau} k'1 \times [A]dt + B0 \approx k'1 \times [A]\tau$$

$$\frac{d[C]}{dt} = k'2 \times [B], \qquad [C] = \int^{\tau} k'2 \times [B]dt + C0 \approx k'2 \times [B]\tau$$

$$\frac{d[D]}{dt} = k'3 \times [C], \qquad [D] = \int^{\tau} k'3 \times [C]dt + D0 \approx k'3 \times [C]\tau$$

Here, B0, C0, and D0 are the initial concentrations of the compounds B, C, and D, and are sufficiently small with respect to the concentration of the precursor substance and can be ignored.

In the case of the target substance o and the related substance p, the quantitative relationship (o)/(p) thereof is given as (o)/(p) = (o) (kjo, koj, (j), $\tau$; j $\neq$ o)/(p) (kjp, kpj, (j), $\tau$; j $\neq$ p). Here, the quantitative relationship (o)/(p) can be expressed as a simple analytical function by the above approximation, or can be numerically expressed by a simple numerical solution. For example, when the target substance o or the related substance p is D, it can be approximated as (o) or (p) = k'3k'2k'1(A)-$\tau^3$, and the quantitative relationship (o)/(p) can be derived using this. Note that as described above, $\tau$ can empirically input the period until the biosynthetic metabolism stops.

The quantitative relationship (o)/(p) between the target substance o and the related substance p can be more easily determined by selecting a related substance of which a content correlation can be approximated by a first order. Examples of a case where the content correlation can be approximated by a first order include a case where a metabolic pathway for generating the target substance o and a metabolic pathway for generating the related substance p stably branch from a common metabolic pathway. When the metabolic pathway between the target substance o and the related substance p stably branch in this way, the content of the compound generated next to the common precursor substance in both metabolic pathways is actually measured to obtain a ratio of the contents, and this ratio can be regarded as the quantitative relationship (o)/(p) between the target substance o and the related substance p. As a further simple example, when it can be approximated that from the precursor substance A, the target substance o is generated with a reaction rate constant kAo, and the related substance p is generated with a reaction rate constant kAp, the quantitative relationship (o)/(p) = kAo/kAp can be derived based on Expression 3.

Fig. 5 shows a part of a simplified apple secondary metabolic pathway which can be approximated by a first order. In the simplified secondary metabolic pathway, a glycolytic pathway which uses D-glucose as a precursor substance and generates phosphoenolpyruvic acid (PEP) branches into a flavonoid biosynthetic pathway and a carotenoid biosynthetic pathway. The metabolic pathway leading to procyanidin, which is the target substance of the present embodiment, is a flavonoid generation pathway using PEP as a precursor substance. The metabolic pathway leading to $\beta$ carotene, which is the related substance of the present embodiment, is a carotenoid biosynthetic pathway using PEP as a precursor substance. In both pathways, the glycolytic pathway is common, and the precursor substance PEP is common.

In a flavonoid synthesis pathway, each precursor is considered to be present in a sufficiently greater amount than the product. Therefore, a relationship between the concentration of procyanidin, which is the target substance of the present embodiment, and the reaction rate can be approximated as in above-described Expression 3.

In addition, it may be considered that a ratio at which the flavonoid biosynthetic pathway and the carotenoid biosynthetic pathway branch from PEP does not change significantly during the growth period. In other words, it may be considered that the pathways stably branch from PEP. Therefore, a quantitative relationship between procyanidin and β carotene can be approximated by a first order. For example, the amounts of 3-dehydroshikimate generated next to PEP on the flavonoid biosynthetic pathway side and pyruvic acid generated next to PEP on the carotenoid biosynthetic pathway side are actually measured by spectroscopic measurement or the like, and the ratio thereof can be approximated to the quantitative relationship (o)/(p) between procyanidin and β carotene. Note that the substance actually measured to take a first order approximation is not limited to the substance generated next to PEP. When the ratio of the contents can be approximated to the quantitative relationship (o)/(p) between procyanidin and β carotene, it is possible to actually measure an intermediate product on the flavonoid biosynthetic pathway side and an intermediate product on the carotenoid biosynthetic pathway side, which are predicted from the reaction equation system, and to take the first order approximation from the ratio of the contents. In this way, in the case of taking the first order approximation from the actual measurement of the intermediate product, the first order approximation with higher accuracy may be taken by calculating the ratio of the contents for a plurality of sets of intermediate products. In addition, not only the intermediate product but also the procyanidin on the flavonoid biosynthetic pathway side is measured by destructive measurement, the amount of β carotene on the carotenoid biosynthetic pathway side is measured by spectroscopic measurement or the like, and the ratio thereof can be approximated to the quantitative relationship between the procyanidin and the β carotene. In addition, a ratio between a reaction rate constant of a rate-limiting reaction in the flavonoid biosynthetic pathway and a reaction rate constant of a rate-limiting reaction in the carotenoid biosynthetic pathway can also be approximated to the quantitative relationship between procyanidin and β carotene.

In the present embodiment, as an example, the above-described first order approximation was employed, and the quantitative relationship (o)/(p) between procyanidin and β carotene was given as a constant from the ratio of the measured amounts of 3-dehydroshikimate and pyruvic acid generated next to PEP. The estimation unit 13 estimates the content of procyanidin, which is the target substance, by multiplying the content of β carotene as the related substance quantified from the measurement result of the Raman spectrum by the measurement unit 12 (related substance quantification unit 122) by the constant value of the quantitative relationship (o)/(p).

Further, the estimation unit 13 compares the content (estimated value) of the target substance estimated by the analysis method according to the present embodiment with the content (measured value) of the target substance separately measured by invasive measurement. For example, a primary correlation is assumed, and a variation between the estimated value and the measured value is verified. Accordingly, the accuracy of the quantitative relationship between the target substance and the related substance can be verified, and the estimation accuracy of the target substance can be determined.

Fig. 6 shows a graph in which the procyanidin content (estimated value) estimated based on the quantitative relationship with the related substance as described above is compared with the procyanidin content (measured value) measured by the chromatography method for the same object. When taking the first order approximation of the plot of the graph, $y = 0.9971x$, and the variation was 0.95. From this, in the apple, the procyanidin amount estimated based on the amount of β carotene and the measured procyanidin amount nearly approximate, which supports the validity of estimating the procyanidin amount from the amount of β carotene.

Fig. 7 shows a flow of a component analyzing method according to the present embodiment.

In step 11, the projection unit 11 generates the measurement light by the light source 111 and projects the measurement light on the object.

In step 12, the measurement unit 12 detects reflected light, scattered light, or emitted light from the object by the detection unit 121, and measures the content of the related substance, which is contained together with the target substance in the object, from the detection result by the related substance quantification unit 122. The details are as described above.

In step 13, the estimation unit 13 estimates the content of the target substance from the measurement result of the content of the related substance measured in step 12 based on the quantitative relationship between the target substance and the related substance derived from the reaction equation system based on the biosynthetic metabolic pathway of the object. The details are as described above.

The component analyzing device 1 according to the present embodiment includes: the projection unit 11 which projects light on an object; the measurement unit 12 which detects reflected light, scattered light, or emitted light from the object and measures a content of a related substance, which is contained together with a target substance in the object, from a detection result; and the estimation unit 13 which estimates a content of the target substance from a measurement result of the content of the related substance, based on a quantitative relationship between the target substance and the related substance derived from a reaction equation system based on a biosynthetic metabolic pathway of the object. According to this, the content of the related substance contained in the object is optically measured, and from the result, the content of the target substance contained in the object is estimated based on the quantitative relationship between the target substance and the related substance. The quantitative relationship is derived from the reaction equation system based on the biosynthetic metabolic pathway of the object. In the biosynthetic metabolic pathway of apple mentioned as an example, the biosynthetic pathway of procyanidin, which is an example of the target substance, and the biosynthetic pathway of cartenoid, which is an example of the related substance, are partially common, and a correlation is observed between the contents thereof, and thus, the carotenoid with a large content is non-invasively measured, and the content of the procyanidin is estimated based on the measurement result, whereby the content of the target substance which is difficult to be non-invasively measured can be detected with high accuracy.

In addition, the component analyzing method according to the present embodiment includes projecting light on an object; detecting reflected light, scattered light, or emitted light from the object and measuring a content of a related substance, which is contained together with a target substance in the object, from a detection result; and estimating a content of the target substance from a measurement result of the content of the related substance, based on a quantitative relationship between the target substance and the related substance derived from a reaction equation system based on a biosynthetic metabolic pathway of the object. According to this, the content of the related substance contained in the object is optically measured, and from the result, the content of the target substance contained in the object is estimated based on the quantitative relationship between the target substance and the related substance. The quantitative relationship is derived from the reaction equation system based on the biosynthetic metabolic pathway of the object. In the biosynthetic metabolic pathway of apple mentioned as an example, the biosynthetic pathway of procyanidin, which is an example of the target substance, and the biosynthetic pathway of cartenoid, which is an example of the related substance, are partially common, and a correlation is observed between the contents thereof, and thus, the carotenoid with a large content is non-invasively measured, and the content of the procyanidin is estimated based on the measurement result, whereby the content of the target substance which is difficult to be non-invasively measured can be detected with high accuracy.

The component analyzing device 1 and the component analyzing method according to the present embodiment are suitable for estimating the content of the target substance that is difficult to be non-invasively measured, but may be used for estimating the content of the target substance which is easy to perform non-invasive measurement. In addition, the object is not limited to apples, and may be any plant such as fruits or grains, and the target substance is also not limited to procyanidin and may be arbitrarily selected.

As described above, according to the present invention, even when a target substance is difficult to be non-invasively measured, for example, since only a trace amount of the target substance is contained in the object, the absorption or reflection spectrum of the target substance overlaps with that of another substance in the object and cannot be spectroscopically measured, or the content of the target substance fluctuates by the influence of a measurement action due to high reactivity, it is possible to perform non-invasive measurement of the related substance which is correlated with the target substance in the biosynthesis of the object, and estimate, from the result of the non-invasive measurement, the content of the target substance, based on the quantitative relationship between the target substance and the related substance derived from the reaction equation system based on the biosynthetic metabolic pathway of the object.

While the present invention has been described by way of the embodiments, the technical scope of the present invention is not limited to the above described embodiments. It is apparent to persons skilled in the art that various alterations or improvements can be made to the above described embodiments. It is also apparent from the described scope of the claims that the embodiments added with such alterations or improvements can be included the technical scope of the present invention.

The operations, procedures, steps, stages, or the like of each process performed by a device, system, program, and method shown in the claims, embodiments, or diagrams can be performed in any order as long as the order is not indicated by "prior to," "before," or the like and as long as the output from a previous process is not used in a later process. Even if the process flow is described using phrases such as "first" or "next" in the claims, embodiments, or diagrams, it does not necessarily mean that the process must be performed in this order.

EXPLANATION OF REFERENCES

1: component analyzing device
11: projection unit
12: measurement unit
13: estimation unit.

## Claims

1. A component analyzing device comprising:

a projection unit which projects light on an object;

a measurement unit which detects reflected light, scattered light, or emitted light from the object and measures a content of a related substance, which is contained together with a target substance in the object, from a detection result; and
an estimation unit which estimates a content of the target substance from a measurement result of the content of the related substance, based on a quantitative relationship between the target substance and the related substance derived from a reaction equation system based on a biosynthetic metabolic pathway of the object.

**2.** The component analyzing device according to claim 1, wherein the quantitative relationship is derived based on a reaction equation system and a reaction rate constant regarding a metabolic pathway leading to each of the target substance and the related substance from a precursor substance common to the target substance and the related substance in the biosynthetic metabolic pathway of the object.

**3.** The component analyzing device according to claim 2, wherein the quantitative relationship is derived by first-order approximation of the reaction equation system regarding the metabolic pathway leading to each of the target substance and the related substance from the precursor substance.

**4.** The component analyzing device according to any one of claims 1 to 3, wherein the related substance is selected from substances that at least partially have a common metabolic pathway with the target substance in the biosynthetic metabolic pathway of the object.

**5.** The component analyzing device according to any one of claims 1 to 4, wherein the related substance is selected from substances, which allow measurement using light and have a large content, among substances contained in the object.

**6.** The component analyzing device according to any one of claims 1 to 5, wherein a metabolic pathway which generates the target substance and a metabolic pathway which generates the related substance stably branch from a common metabolic pathway in the biosynthetic metabolic pathway of the object.

**7.** The component analyzing device according to any one of claims 1 to 6, wherein the estimation unit further verifies an estimated content of the target substance by comparison with a measured content of the target substance.

**8.** The component analyzing device according to any one of claims 1 to 7, wherein

the object is a plant including a fruit and a vegetable,
the target substance is procyanidin, and
the related substance is a carotenoid.

**9.** A component analyzing method comprising:

projecting light on an object;
detecting reflected light, scattered light, or emitted light from the object and measuring a content of a related substance, which is contained together with a target substance in the object, from a detection result; and
estimating a content of the target substance from a measurement result of the content of the related substance, based on a quantitative relationship between the target substance and the related substance derived from a reaction equation system based on a biosynthetic metabolic pathway of the object.

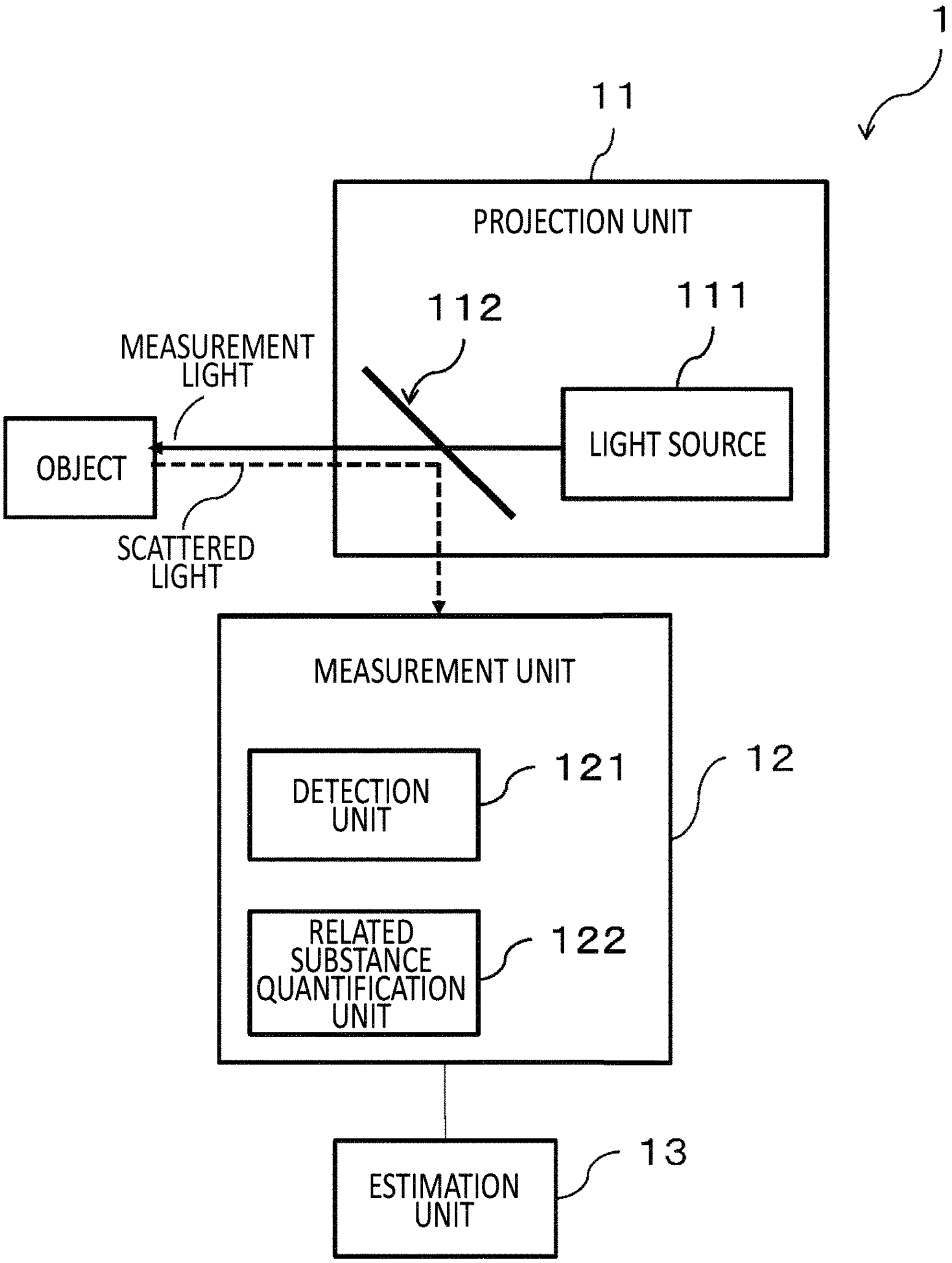
1
11
PROJECTION UNIT
112
111
MEASUREMENT LIGHT
OBJECT
LIGHT SOURCE
SCATTERED LIGHT
MEASUREMENT UNIT
DETECTION UNIT
121
12
RELATED SUBSTANCE QUANTIFICATION UNIT
122
ESTIMATION UNIT
13

FIG.1

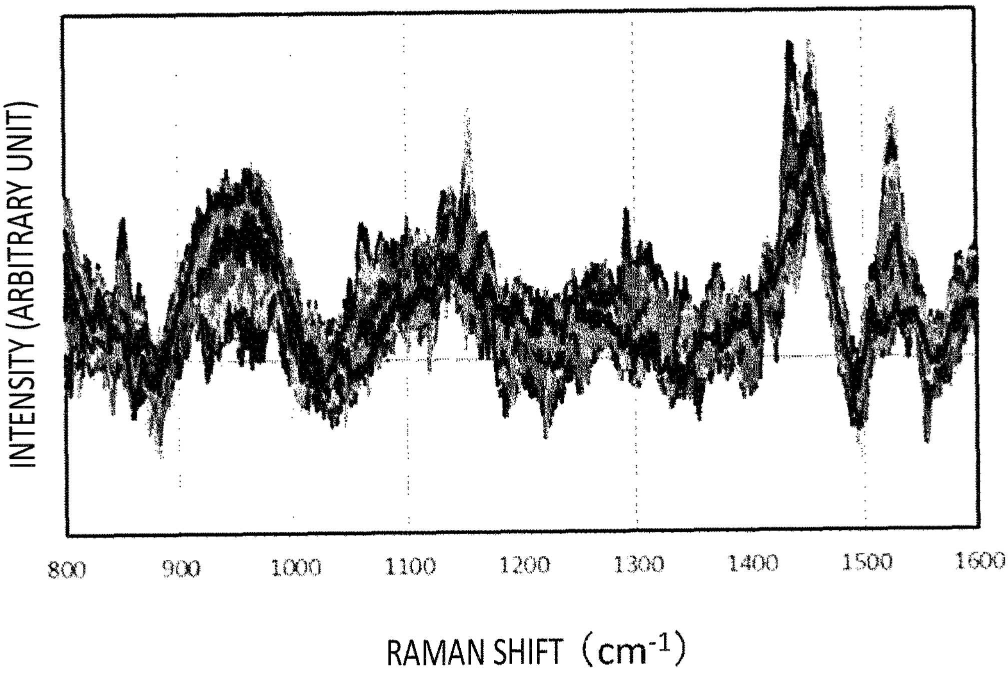
INTENSITY (ARBITRARY UNIT)
800
900
1000
1100
1200
1300
1400
1500
1600
RAMAN SHIFT (cm-1)

*FIG.2*

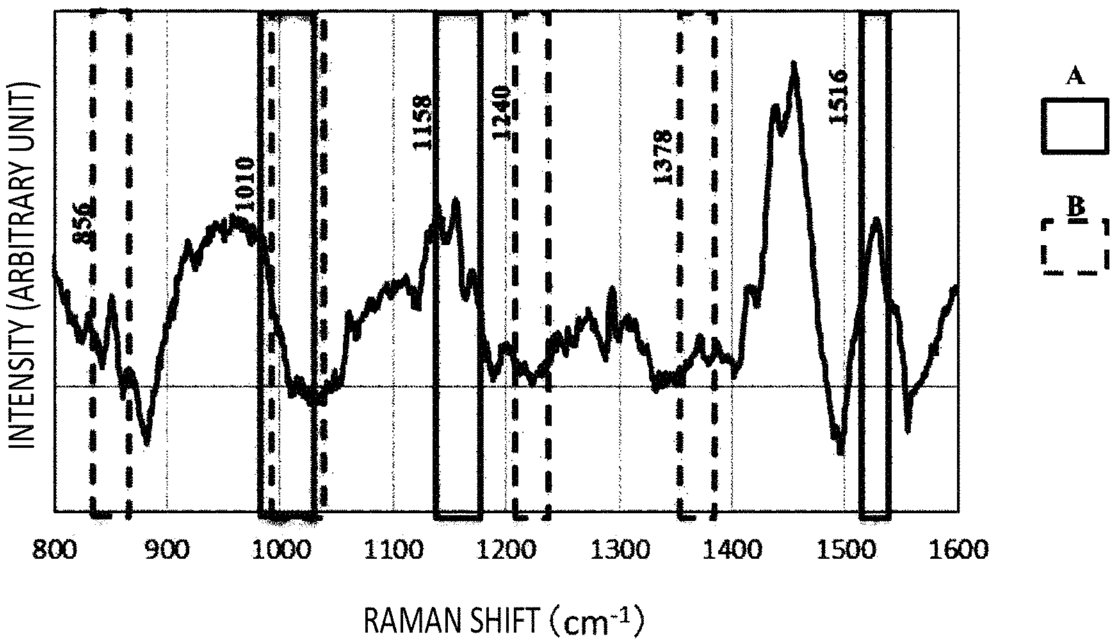
INTENSITY (ARBITRARY UNIT)
856
1010
1158
1240
1378
1516
A
B
800
900
1000
1100
1200
1300
1400
1500
1600
RAMAN SHIFT (cm-1)

FIG.3

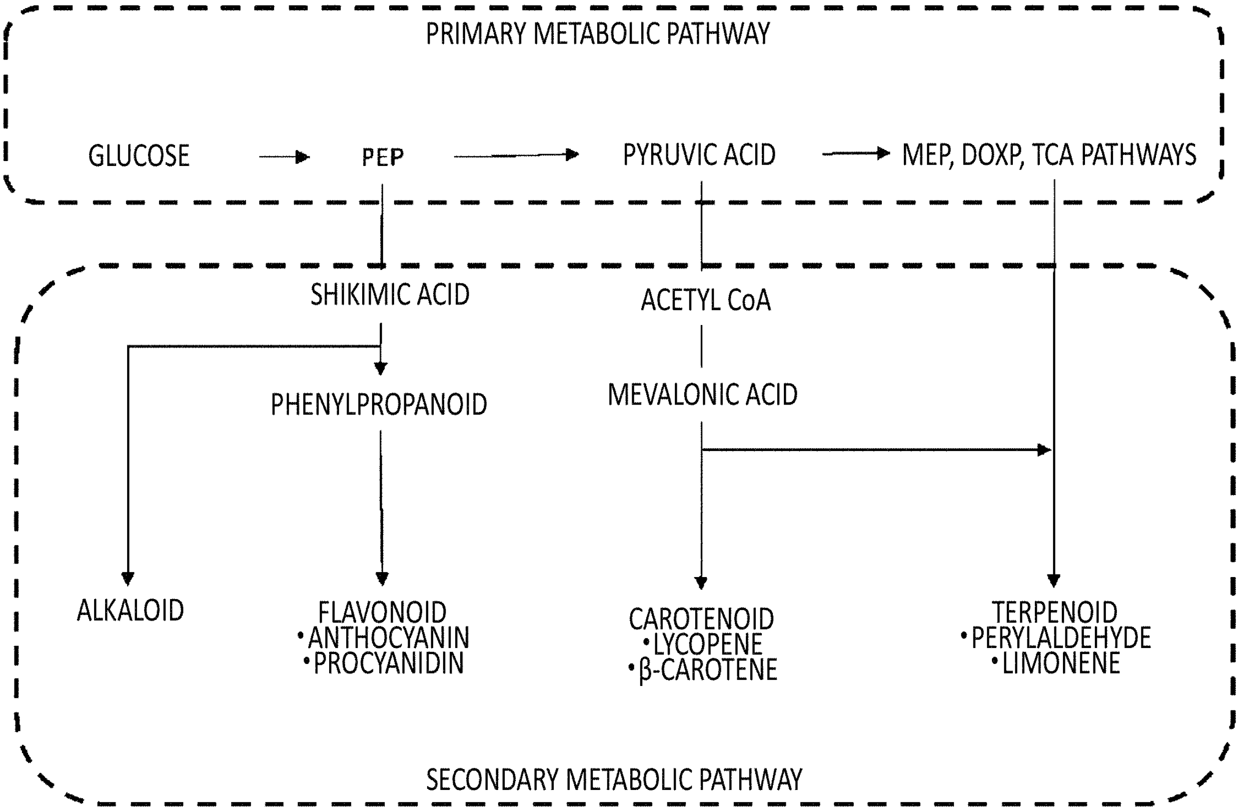
PRIMARY METABOLIC PATHWAY
GLUCOSE
PEP
PYRUVIC ACID
MEP, DOXP, TCA PATHWAYS
SHIKIMIC ACID
ACETYL CoA
PHENYLPROPANOID
MEVALONIC ACID
ALKALOID
FLAVONOID
•ANTHOCYANIN
•PROCYANIDIN
CAROTENOID
•LYCOPENE
•β-CAROTENE
TERPENOID
•PERYLALDEHYDE
•LIMONENE
SECONDARY METABOLIC PATHWAY

*FIG.4*

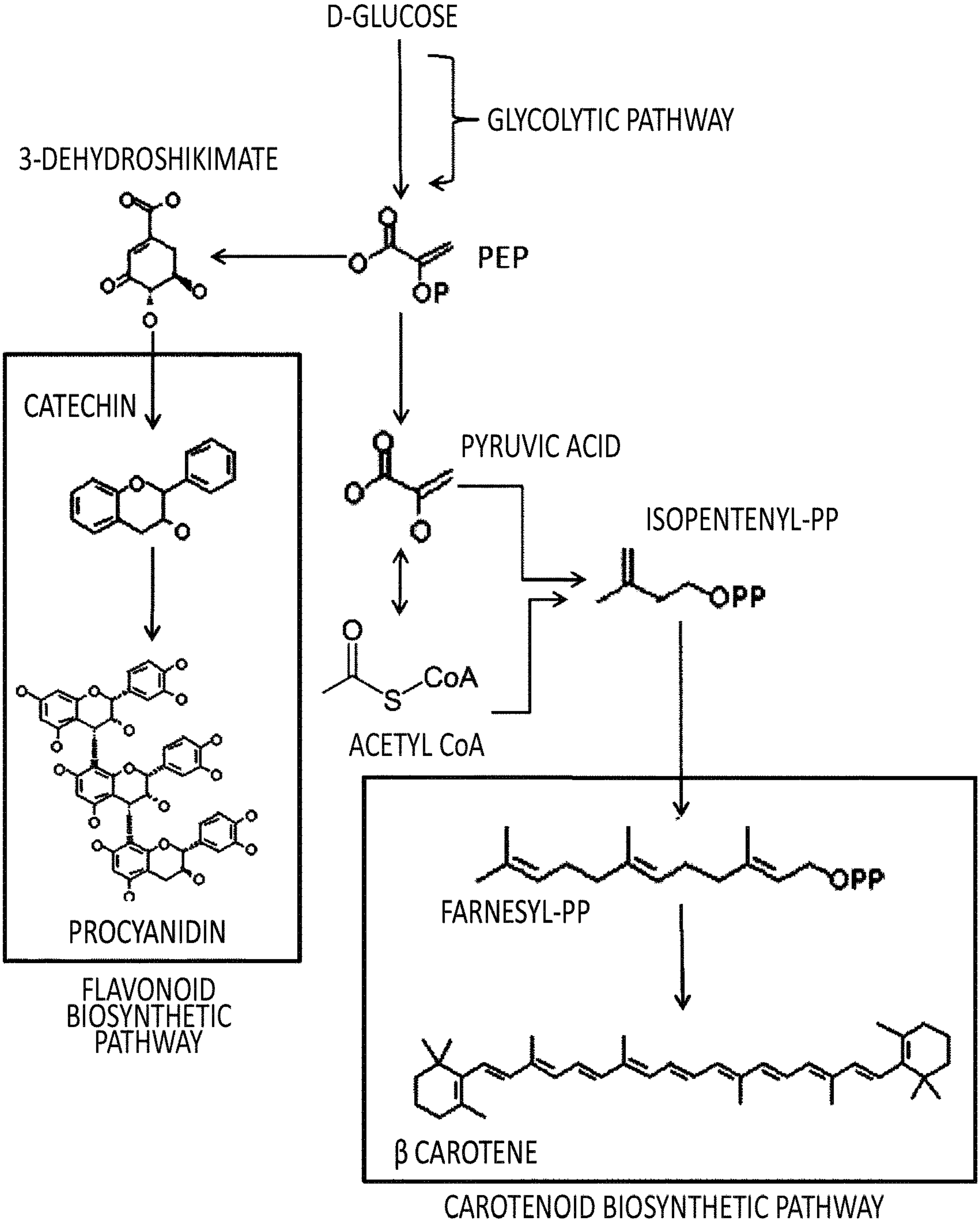
D-GLUCOSE
GLYCOLYTIC PATHWAY
3-DEHYDROSHIKIMATE
PEP
CATECHIN
PYRUVIC ACID
ISOPENTENYL-PP
ACETYL CoA
PROCYANIDIN
FLAVONOID BIOSYNTHETIC PATHWAY
FARNESYL-PP
β CAROTENE
CAROTENOID BIOSYNTHETIC PATHWAY

*FIG.5*

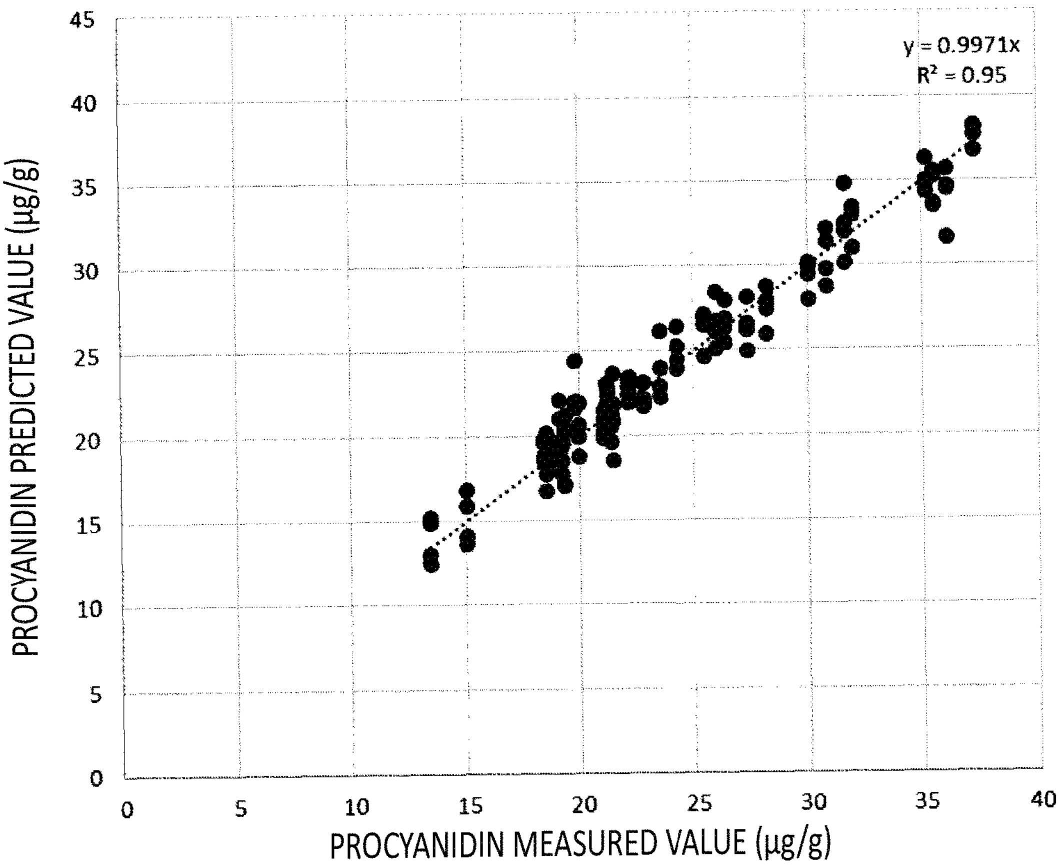
y = 0.9971x
R² = 0.95
PROCYANIDIN PREDICTED VALUE (µg/g)
0
5
10
15
20
25
30
35
40
45
PROCYANIDIN MEASURED VALUE (µg/g)
0
5
10
15
20
25
30
35
40

*FIG.6*

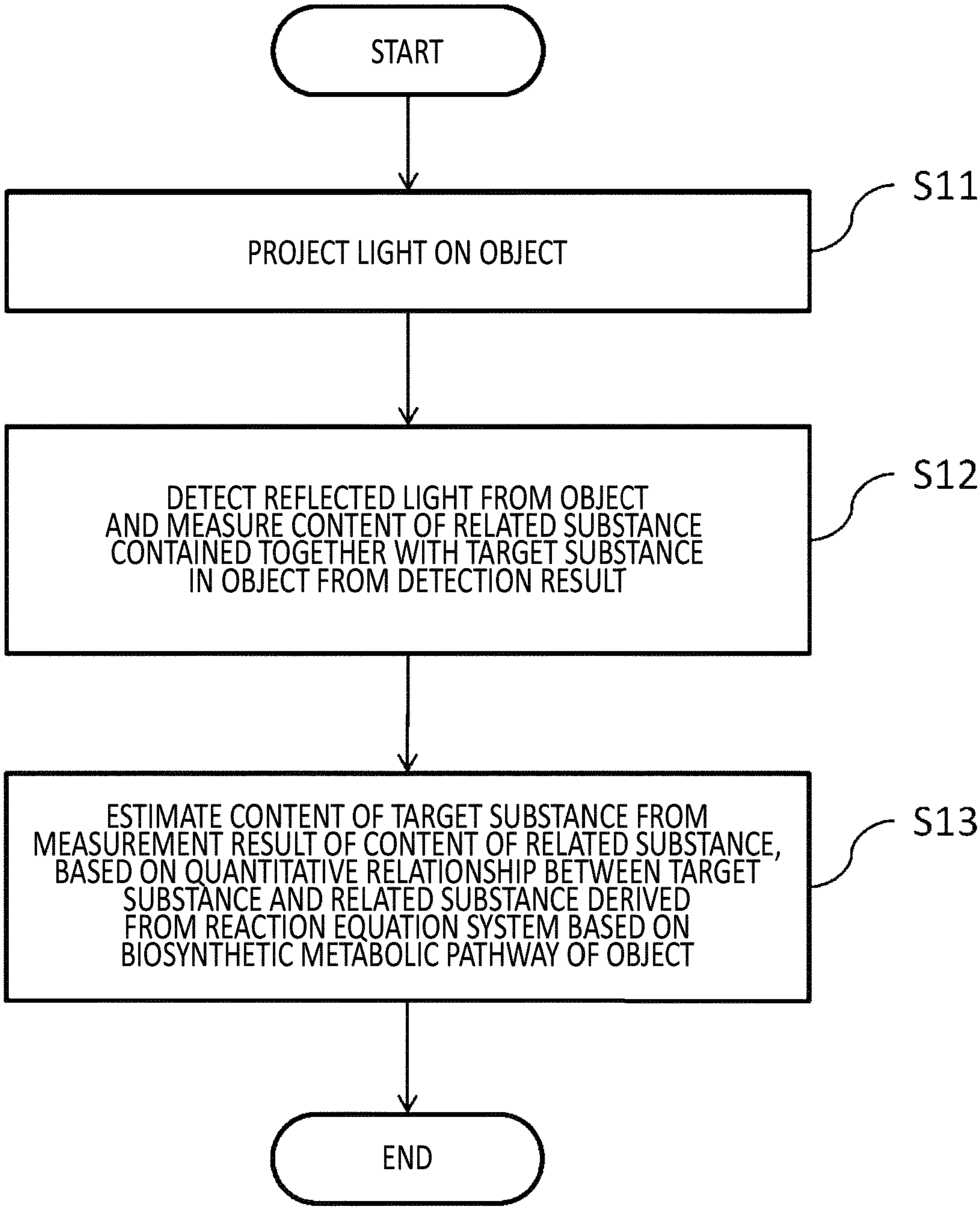
START
S11
PROJECT LIGHT ON OBJECT
S12
DETECT REFLECTED LIGHT FROM OBJECT AND MEASURE CONTENT OF RELATED SUBSTANCE CONTAINED TOGETHER WITH TARGET SUBSTANCE IN OBJECT FROM DETECTION RESULT
S13
ESTIMATE CONTENT OF TARGET SUBSTANCE FROM MEASUREMENT RESULT OF CONTENT OF RELATED SUBSTANCE, BASED ON QUANTITATIVE RELATIONSHIP BETWEEN TARGET SUBSTANCE AND RELATED SUBSTANCE DERIVED FROM REACTION EQUATION SYSTEM BASED ON BIOSYNTHETIC METABOLIC PATHWAY OF OBJECT
END

*FIG.7*

**INTERNATIONAL SEARCH REPORT**

International application No.
**PCT/JP2022/030901**

**A. CLASSIFICATION OF SUBJECT MATTER**

***G01N 21/65***(2006.01)i; ***G01N 21/27***(2006.01)i; ***G01N 21/64***(2006.01)i
FI: G01N21/65; G01N21/27 B; G01N21/64 Z

According to International Patent Classification (IPC) or to both national classification and IPC

**B. FIELDS SEARCHED**

Minimum documentation searched (classification system followed by classification symbols)

G01N21/00-21/958

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | JP 2017-512996 A (METANOMICS GMBH) 25 May 2017 (2017-05-25) | 1-9 |
| A | JP 2017-151063 A (THE UNIVERSITY OF TOKYO) 31 August 2017 (2017-08-31) | 1-9 |
| A | WO 2021/049539 A1 (THE UNIVERSITY OF TOKYO) 18 March 2021 (2021-03-18) | 1-9 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 October 2022** | **08 November 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/030901**

| Patent document cited in search report | Publication date (day/month/year) | Patent family member(s) | Publication date (day/month/year) |
|---|---|---|---|
| JP 2017-512996 A | 25 May 2017 | US 2018/0217125 A1 | |
| | | WO 2015/145387 A1 | |
| | | EP 3123174 A1 | |
| | | CA 2941979 A1 | |
| | | AU 2015237804 A1 | |
| | | KR 10-2016-0146727 A | |
| | | CN 106415274 A | |
| JP 2017-151063 A | 31 August 2017 | (Family: none) | |
| WO 2021/049539 A1 | 18 March 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6323749 B


### Non-patent literature cited in the description


- **H. HIGASHIO.** *Proceedings of vegetable and tea science,* 2004
- **W. KOWALLIK et al.** *Planta,* 1976, vol. 128, 11
- **MASAYASU M. et al.** *Hort.Res. (Japan,* 2015, vol. 14 (4), 391-396